# EUROPÄISCHE PATENTSCHRIFT

(11) **EP 0 352 540 B1**
(45) Veröffentlichungstag und Bekanntmachung des Hinweises auf die Patenterteilung: **01.12.1993**
(21) Anmeldenummer: 89112629.4
(22) Anmeldetag: 11.07.1989
(51) Int. Cl.: A61M 1/14

(54) **Verschlusskappe für Dialysatoren**
Closure cap for dialysers
Capuchon de fermeture pour dialyseurs

(30) Priorität: 28.07.1988 DE 3825573
(43) Veröffentlichungstag der Anmeldung: 31.01.1990
(73) Patentinhaber: Fresenius AG, 61350 Bad Homburg (DE)
(72) Erfinder: Mathieu, Bernd, Dr. Dipl.-Chem., D-6683 Spiesen-Elversberg (DE); Rinck, Michael, Dr., D-6670 St. Ingbert-Hassel (DE); Schnur, Jürgen, D-6692 Oberthal (DE); Selzer, Dieter, D-6698 Namborn (DE); Weber, Wolfram, D-6683 Spiesen-Elversberg (DE); Heilmann, Klaus, D-6680 Neunkirchen (DE)
(74) Vertreter: Fuchs Mehler Weiss

(56) Entgegenhaltungen:
- US-A- 4 375 413

## Beschreibung

Die Erfindung betrifft eine Verschlußkappe für Dialysatoren nach dem Oberbegriffs des Anspruchs 1 sowie einen mit einer derartigen Verschlußkappe versehenen Dialysator.

Dialysatoren müssen nach ihrer eigentlichen Herstellung vor ihrer Lagerung sterilisiert werden. Hierbei muß bei der Auslegung des Sterilisationsverfahrens als sog. In-line-Verfahren jeder offene Kontakt mit der Außenluft nach der Durchführung des Sterilisationsschrittes vermieden werden. Aus diesem Umstand heraus ergibt sich die Notwendigkeit des keimdichten Abschließens des Dialysators vor dessen Abnahme vom Sterilisationsstand.

Eine dem Oberbegriff des Anspruchs 1 entsprechende Verschlußkappe weist ein erstes Kappenteil auf, das mit zwei Schenkeln in eine kreisringförmige Ausnehmung eines Dialysatorflansches eingeführt wird, und an dem ein zweites Kappenteil befestigt ist, das zwischen einer Offen- und einer Schließstellung bewegbar ist. Dieses zweite Kappenteil weist eine Schließplatte auf, die über eine Gelenkverbindung mit dem ersten Kappenteil verbunden ist und an der ein Führungsteil vorgesehen ist, das in der Schließstellung in einen Anschlußflansch des ersten Kappenteiles eingreift.

Es hat sich jedoch gezeigt, daß die gattungsgemäße Verschlußkappe nicht ausreichend keimdicht ist. Ferner ermöglicht es die bekannte Verschlußkappe nicht, im Zuge der Sterilisation auch die Flanschinnenflächen durch das Sterilisationsmedium zu entkeimen. Schließlich ist die gattungsgemäße Verschlußkappe insoweit nachteilig, als sie nicht für die Verwendung bei In-line-Sterilisationsverfahren für Dialysatoren geeignet ist.

Aus der US-A-43 75 413 ist ferner eine Spülvorrichtung für den Wiedergebrauch eines Dialysators bekannt, bei der eine an einem Konnektor befindliche Spüldüse in den Blutzulaufraum des Dialysators eingeführt wird. Der Konnektor selbst weist kein Verschlußglied auf und kann im übrigen nicht vom Zulaufschlauch abgetrennt werden.

Es ist daher Aufgabe der vorliegenden Erfindung, eine Verschlußkappe für Dialysatoren nach dem Oberbegriff des Anspruchs 1 zu schaffen, die für die Verwendung bei In-line-Sterilisationsverfahren für Dialysatoren geeignet ist, die einen keimdichten Abschluß sowohl blut- als auch dialysatseitig ermöglicht und die darüber hinaus die gleichzeitige Entkeimung der Flanschinnenflächen im Zuge der Sterilisation erlaubt.

Die Lösung dieser Aufgabe erfolgt durch die Merkmale des Anspruchs 1.

Durch die erfindungsgemäße Verschlußkappe wird ein keimdichter Sterilverschluß bei aufgesetzter Verschlußkappe in Schließstellung möglich gemacht, der das Einlegen der außen unsterilen Dialysatoren in eine unsterile Umverpackung ermöglicht. Hierbei erfolgt bei einer ersten Ausführungsform eine Abdeckung, bei der die Außenhülse den Dialysatorflansch steril dichtend außen übergreift, während bei einer zweiten Ausführungsform die sterile Dichtung über eine elastische Dichteinrichtung erfolgt.

Durch die bauartbedingte Anschlußmöglichkeit an eine In-line-Sterilisationsapparatur ist es möglich, LF-geschützte aseptische Verschlußstationen zu vermeiden.

Ferner erhöht die Sterilisation der Flanschinnenseiten des Dialysators die mikrobiologische Sicherheit.

Die in-line-sterilisierbare Verschlußkappe gemäß vorliegender Erfindung ist vorteilhafterweise zur Vormontage in geöffnetem Zustand geeignet und verbleibt während und nach der Sterilisation am Dialysator. Hierbei werden sämtliche Kappeninnenseiten mitsterilisiert, und es wird möglich, die Verschlußkappe vor Entnahme des Dialysators aus dem Produktionsprozeß automatisch zu verschließen, so daß ein keimdichter Abschluß sowohl blut- als auch dialysatseitig gewährleistet ist.

Dadurch wird jeder offene Kontakt mit der Außenluft nach der Durchführung der Sterilisation vermieden, so daß der Dialysator keimsicher nach außen abgedichtet ist, wobei neben einer automatischen Verriegelung auch eine Verriegelung von Hand möglich ist.

Durch Verwendung der erfindungsgemäßen Verschlußkappe ist es zur Vermeidung von Verschleppung von Kontaminationen möglich, eine am Dialysatorflansch vorgesehene Verbindungseinrichtung, beispielsweise in Form eines Schraubanschlusses, vollständig keimfrei zu halten.

Durch die Konstruktion der erfindungsgemäßen Verschlußkappe wird es vorteilhafterweise möglich, alle Gleitflächen und den Kontaktbereich zum Dialysatorflansch zu sterilisieren, wobei die Sterilisierung des Konnektoransatzes am Flansch ebenfalls möglich ist.

Als ein weiterer Vorteil der erfindungsgemäßen Verschlußkappe ist hervorzuheben, daß sie für alle Arten von Sterilisationsverfahren mit allen derzeit gängigen Sterilisationsmitteln, wie Heiß- und Kaltsterilisationsmedien geeignet ist. Des weiteren kann die erfindungsgemäße Verschlußkappe sowohl bei Dialysatoren verwendet werden, die nach der Sterilisation getrocknet werden, als auch bei solchen, die beispielsweise mit Kochsalzlösung gefüllt werden.

Darüber hinaus ist die erfindungsgemäße Verschlußkappe sowohl auf der Blutseite des Dialysators wie auch auf der Dialysatseite desselben zu verwenden.

Die Unteransprüche haben vorteilhafte Weiterbildungen der Erfindung zum Inhalt. Insbesondere ist es möglich, die Arretiereinrichtung als Originalitätsverschluß auszulegen, der mit exakt definierten Sollbruchstellen versehen sein kann.

Alles in allem ermöglicht die Ausführung der erfindungsgemäßen Verschlußkappe die Durchführung von In-line-Sterilisationsverfahren mit flüssigen und gas-/dampfförmigen Medien, wobei die Dialysatoren sowohl flüssigkeitsgefüllt als auch trocken zur Auslieferung kommen können.

Weitere Einzelheiten, Merkmale und Vorteile der Erfindung ergeben sich aus der nachfolgenden Beschreibung von Ausführungsbeispielen anhand der Zeichnung:
Es zeigt:
- Fig. 1: eine geschnittene auseinandergezogene Darstellung der erfindungsgemäßen Verschlußkappe vor Montage am Dialysatorflansch,
- Fig. 2: eine geschnittene Darstellung der Verschlußkappe gemäß Fig. 1 in Offenstellung und im am Dialysator montierten Zustand,
- Fig. 3: eine der Fig. 2 entsprechende Darstellung der Verschlußkappe, jedoch in Schließstellung,
- Fig. 4: eine der Fig. 1 entsprechende Darstellung einer Ausführungsform der Verschlußkappe, die zur Verwendung auf der Dialysatseite vorgesehen ist,
- Fig. 5: eine der Fig. 2 entsprechende Darstellung der Verschlußkappe gemäß Fig. 4,
- Fig. 6: eine der Fig. 3 entsprechende Darstellung der Verschlußkappe gemäß den Figuren 4 und 5,
- Fig. 7: eine der Fig. 2 entsprechende Darstellung einer zweiten Ausführungsform einer Verschlußkappe für die Blutseite von Dialysatoren,
- Fig. 8: eine der Fig. 5 entsprechende Darstellung der zweiten Ausführungsform einer Verschlußkappe für die Dialysatseite,
- Fig. 9: einen Längsschnitt durch ein stopfenartiges Kappenteil der zweiten Ausführungsform,
- Fig. 10: eine Seitenansicht des Kappenteils gemäß Fig. 9 und
- Fig. 11: eine Stirnansicht des Kappenteiles gemäß Figuren 9 und 10.

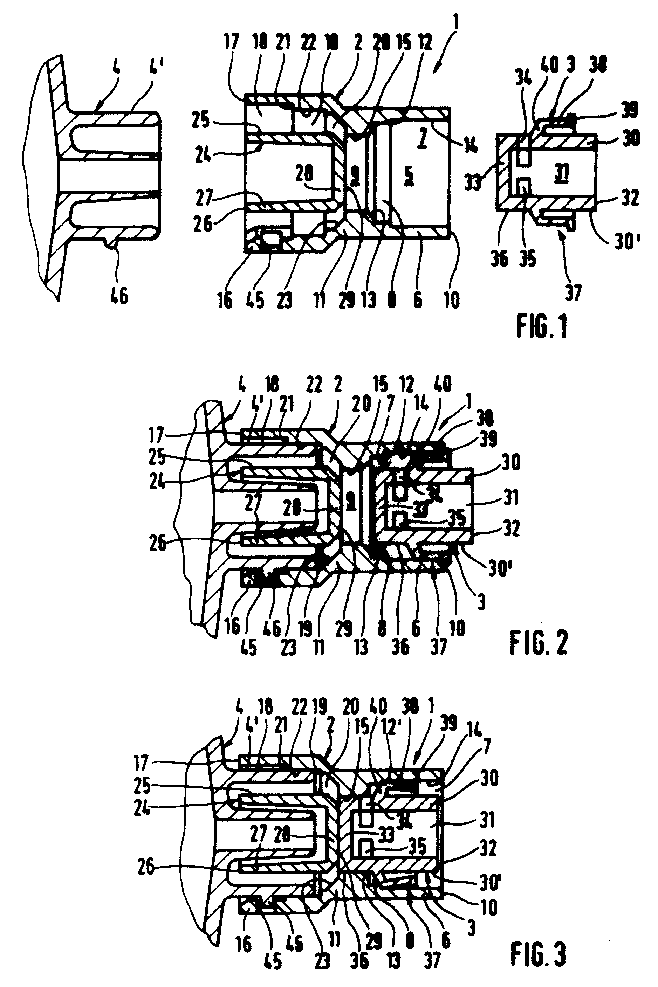

In den Figuren 1 bis 3 ist eine erfindungsgemäße Verschlußkappe 1 für die Blutseite von Dialysatoren dargestellt.

Die Verschlußkappe 1 weist ein erstes Kappenteil 2 und ein zweites stopfenartiges Kappenteil 3 auf.

Das erste Kappenteil 2 ist im wesentlichen rohrförmig ausgebildet, wobei eine bevorzugte Ausführungsform kreiszylindrisch ist. Ferner weist das erste Kappenteil einen ersten Abschnitt 6 und einen zweiten Abschnitt 16 auf. Im ersten Abschnitt 6, dessen Außenabmessung geringer als diejenige des zweiten Abschnittes 16 ist, ist ein Aufnahmeraum 5 für das zweite Kappenteil 3 angeordnet. Ferner bildet der erste Abschnitt 6 ein rückwärtiges Teil des ersten Kappenteils 2, der die Anschlußstelle an einer In-line-Sterilisationsapparatur bildet und hierzu erforderlichenfalls mit geeigneten Anschlußeinrichtungen versehen sein kann.

Der Aufnahmeraum 5 weist drei aufeinanderfolgende Teilkammern 7, 8, 9 auf. Diese Teilkammern 7, 8, 9 sind von einer offenen Stirnseite 10 her mit abnehmenden Durchmessern zu einem der Stirnseite 10 gegenüberliegenden Endbereich 11 des ersten Abschnittes 6 in diesem angeordnet. Hierbei ist zwischen der ersten Teilkammer 7, die an die Stirnseite 10 angrenzt und die den größten Durchmesser bzw. die größte Innenabmessung aufweist, und der zweiten Teilkammer 8 eine im wesentlichen senkrecht zur Längsachse des ersten Kappenteiles 2 nach innen verlaufende Stufe 12 angeordnet.

Die zweite Teilkammer 8 und die dritte Teilkammer 9, die den kleinsten Innendurchmesser bzw. die kleinste Innenabmessung aufweist, sind über einen kegelstumpfförmigen Zwischenabsatz 13 miteinander verbunden, dessen Innenfläche einen stetigen Übergang von der Innenfläche der zweiten Teilkammer 8 zu der Innenfläche 15 der dritten Teilkammer 9 schafft.

Die Innenfläche 14 der ersten Teilkammer 7 ist glattflächig ausgebildet und dient als Führungs- und Haltefläche für das zweite Kapenteil 3, das im folgenden näher beschrieben werden wird. Die Innenfläche 15 der dritten Teilkammer 9 bildet ebenfalls eine Führungsfläche für eine entsprechende Fläche des Kappenteiles 3, worauf auch im folgenden näher eingegangen werden wird.

Der zweite Abschnitt 16 des ersten Kappenteiles 2 ist mit dem ersten Abschnitt 6 verbunden, wobei der zweite Abschnitt 16 an einen Endbereich 11 des ersten Abschnittes 6 über eine kegelstumpfförmige Verbindungsfläche anschließt. Der zweite Abschnitt 16 ist an seiner der Stirnfläche 10 gegenüberliegenden Stirnseite 17 offen, wobei sich an die Stirnseite 17 nach innen drei Teilräume 18, 19 und 20 anschließen. Hierbei weist der unmittelbar an die Stirnseite 17 anschließende Teilraum 18 einen größeren Innendurchmesser bzw. eine größere lichte Weite auf, als der mittlere Teilraum 19. Die Teilräume 18 und 19 sind über eine Stufe 21 miteinander verbunden, die sich im wesentlichen senkrecht zur Längsachse des ersten Kappenteiles 2 nach innen erstreckt.

Wie aus Fig. 2 ersichtlich ist, bildet die Innenfläche 22 des zweiten Teilraumes 19 eine Führungs- und Dichtfläche für einen Dialysatorflansch 4. Hierbei liegt die Innenfläche 22 des zweiten Teilraumes 19 auf dem entsprechenden, nach außen weisenden Wandbereich 4′ eines äußeren umlaufenden Konnektoransatzes auf. Dadurch wird der vom Dialysatorflansch 4 und den Teilräumen 18 bis 20 begrenzte Innenraum abgedichtet.

Der dritte Teilraum 20 des zweiten Abschnittes 16 weist eine kegelstumpfförmige Innenfläche 23 auf, die einen stetigen Übergang zwischen dem zweiten Teilraum 19 und der dritten Teilkammer 9 des ersten Abschnittes 6 schafft. Wie aus Fig. 1 ersichtlich ist, wird durch diese Anordnung eine Strömungsverbindung geschaffen, die durch den gesamten Innenraum des ersten Kappenteiles 2 von der einen Stirnseite 10 bis zur anderen Stirnseite 17 hindurch verläuft.

Wie ferner aus Fig. 1 ersichtlich ist, ist innerhalb der Teilräume 18 bis 20 konzentrisch zum zweiten Abschnitt 16 ein topfförmiges Einsatzteil 24, beispielsweise durch radial nach außen weisende Streben, festgelegt. Die Außenfläche 25 des Einsatzteiles 24 begrenzt mit den Innenflächen der Teilräume 18 bis 20 einen freien Strömungsdurchgang.

Im einzelnen weist das Einsatzteil 24 eine offene Stirnseite 26 auf, die vorzugsweise in einer Ebene mit der offenen Stirnseite 17 des zweiten Abschnittes 16 liegt. Es ist jedoch auch denkbar, daß die Stirnseiten 26 und 17 in unterschiedlichen Ebenen angeordnet sind.

Das Einsatzteil 24 weist ferner eine vorzugsweise kreiszylindrische Begrenzungswand 27 auf, die konzentrisch um die Längsachse des ersten Kappenteiles 2 verläuft. An der der offenen Stirnseite 26 gegenüberliegenden Seite ist diese Begrenzungswand 27 vorzugsweise einstückig mit einer Anschlußwand 28 verbunden, die sich senkrecht zur Längsachse des ersten Kappenteiles 2 erstreckt. Die Abschlußwand 28 weist auf ihrer zur Teilkammer 9 hinweisenden Seite eine Außenfläche 29 auf, die ebenfalls vorzugsweise senkrecht zur Längsachse des Kappenteiles 2 angeordnet ist.

Wie ferner aus der Fig. 1 ersichtlich ist, ist in der Wand des zweiten Abschnittes 16 eine Ausnehmung 45 angeordnet, die die Wand durchgreift. Diese Ausnehmung 45 dient zur Festlegung des zweiten Kappenteiles 2 am Dialysatorflansch 4, der zu diesem Zweck einen Rastvorsprung 46 aufweist, der bei der dargestellten Ausführungsform radial nach außen vorspringt. Die Raststellung kann aus den Figuren 2 und 3 im einzelnen entnommen werden. Um diese Raststellung einzunehmen, sind die entsprechenden Teile des Dialysatorflansches 4 und des zweiten Kappenteiles 2 elastisch federnd ausgebildet und mit entsprechenden Auflaufschrägen versehen, die im einzelnen aus der Darstellung in Fig. 1 entnehmbar sind. Anstelle einer derartigen Schnapp-Rast-Verbindung ist ebenfalls das Vorsehen einer Gewindeverbindung, eines Bajonettverschlusses oder ähnlicher Verriegelungsvorrichtungen möglich.

In Fig. 1 ist ferner im einzelnen die Ausbildung des zweiten Kappenteiles 3 dargestellt. Das zweite Kappenteil 3 ist topfförmig ausgebildet und weist hierzu eine um die Längsachse verlaufende Wand 30 auf, die einen Innenraum 31 begrenzt. Die Wand 30 ist vorzugsweise kreiszylindrisch ausgebildet. Der Innenraum 31 ist an einer Stirnseite 32 offen, wobei der dortige rückwärtige Teil 30′ der Wand im Bedarfsfalle den Anschlußabschnitt für die In-line-Sterilisationsapparatur bilden kann. Dieser Teil kann dann mit geeigneten Anschlußeinrichtungen versehen sein. An der gegenüberliegenden Stirnseite ist der Innenraum 31 von einer im wesentlichen senkrecht zur Längsachse angeordneten Abschlußwand 33 geschlossen.

Die Wand 30 weist im Beispielsfalle 2 Verbindungsöffnungen 34 und 35 auf, die vom Innenraum 31 bis zur Außenwandfläche 36 der Wand 30 verlaufen, so daß sie eine Strömungsbildung vom Außenbereich des zweiten Kappenteiles 3 zu seinem Innenraum 31 schaffen.

Ferner ist aus Fig. 1 die Anordnung einer Führungs- und Halteeinrichtung 37 auf der Außenwandfläche 36 der Wand 30 ersichtlich. Im Beispielsfalle weist die Führungs- und Halteeinrichtung 37 einen Führungsring 38 auf, der konzentrisch um die Außenwandfläche 36 in einem gewissen Abstand herum verläuft. Der Führungsring 38 ist an seinem der Stirnseite 32 zugewandten Ende mit einem Anschlagring 39 versehen, der sich radial nach außen vom Führungsring 38 aus erstreckt und somit einen nach außen vorspringenden Rand bildet. An seinem anderen Ende ist der Führungsring 38 über einen bezüglich der Längsachse schräg angeordneten Bund 40 mit der Wand 30 verbunden. Vorzugsweise stellt diese Anordnung eine einstückige Verbindung dar.

Die zuvor beschriebene Verschlußkappe 1 ist in Fig. 2 in ihrer Offenstellung dargestellt. In dieser Position ist das zweite Kappenteil 3 in den Aufnahmeraum 5 des ersten Kappenteiles 1 eingeschoben worden. Wie Fig. 2 verdeutlicht, liegt in dieser Stellung die Außenfläche des Führungsringes 38 an der Innenfläche 14 der ersten Teilkammer 7 an. Ferner liegt der Anschlagring 39 des Führungsringes 38 an der Stirnfläche der Stirnseite 10 des ersten Kappenteiles 2 an und begrenzt somit die axiale Lage des zweiten Kappenteiles 3 innerhalb des Aufnahmeraumes 5 des ersten Kappenteiles 2. Das erste Kappenteil 2 ist in der in Fig. 2 dargestellten Position mit dem Dialysatorflansch 4 in der zuvor beschriebenen Art und Weise verbunden, wobei die Dichtflächen 4′ und 22 den gebildeten Raum zwischen dem Dialysatorflansch und dem zweiten Abschnitt 16 des ersten Kappenteiles 2 abdichten.

Wie Fig. 2 ferner verdeutlicht, ist durch die axial begrenzte Lage des zweiten Kappenteiles 3 innerhalb des Aufnahmeraumes 5 ein freier Durchtritt des Sterilisationsmediums durch die gesamte Anordnung gewährleistet. Mithin ist es bei der Sterilisation möglich, sämtliche Teile und Gleitflächen der in Fig. 2 gezeigten Anordnung vollständig zu sterilisieren und mithin völlig keimfrei zu machen. Dies macht es möglich, die erfindungsgemäße Verschlußkappe bei einer sog. In-line-Sterilisation zu verwenden, was bedeutet, daß die Sterilisation in der Herstellungslinie des Dialysators erfolgt.

Nach Beendigung der Sterilisation wird das zweite Kappenteil 3 in seine in Fig. 3 verdeutlichte Schließstellung, vorzugsweise automatisch, bewegt. Hierzu wird das zweite Kappenteil 3 entlang der Längsachse in Richtung auf das Einsatzteil 24 zu gedrückt. Fig. 3 zeigt, daß sich bei dieser Bewegung der elastisch federnde Führungsring 38 auf die Wand 30 zu verformt, so daß ein Einschieben des zweiten Kappenteiles 3 in den Aufnahmeraum 5 möglich ist. Das Einschieben wird so lange fortgesetzt, bis die Außenfläche der Abschlußwand 33 des zweiten Kappenteiles 3 in dichtende Anlage an der Außenfläche 29 des Einsatzteiles 24 gelangt. Hierbei übernimmt die Innenfläche 15 der dritten Teilkammer 9 eine Führungs- und Dichtungsfunktion. Die in Fig. 3 dargestellte Schließ- bzw. Dichtstellung des zweiten Kappenteiles 3 innerhalb des ersten Kappenteiles 2 wird durch die Anlage des Anschlagringes 39 an der Innenfläche 14 gesichert, da der Führungsring 38 aufgrund seiner elastisch federnden Ausbildung den Anschlagring 39 gegen die Innenfläche 14 drückt. Dabei ist es möglich, die zuvor genannten Teile so auszulegen, daß das einen Stopfen bildende zweite Kappenteil 3 nicht mehr in seine Offenstellung bewegt werden kann. In dieser Stellung kann der Dialysator, der aufgrund der Verschlußkappe 1 keimfrei abgedichtet ist, dem Sterilisationsapparat entnommen werden, wobei durch die als Bajonett-, Schnapp- oder Schraubverbindung ausgebildete Arretierungseinrichtung ein Lösen der Verschlußkappe 1 vom Sterilisator, zum Beispiel in der unsterilen Verpackung, sicher verhindert wird.

Wie zuvor bereits erwähnt, handelt es sich bei der in den Figuren. 1 bis 3 dargestellten Verschlußkappe 1 um die Ausführungsform für die Blutseite des Dialysators.

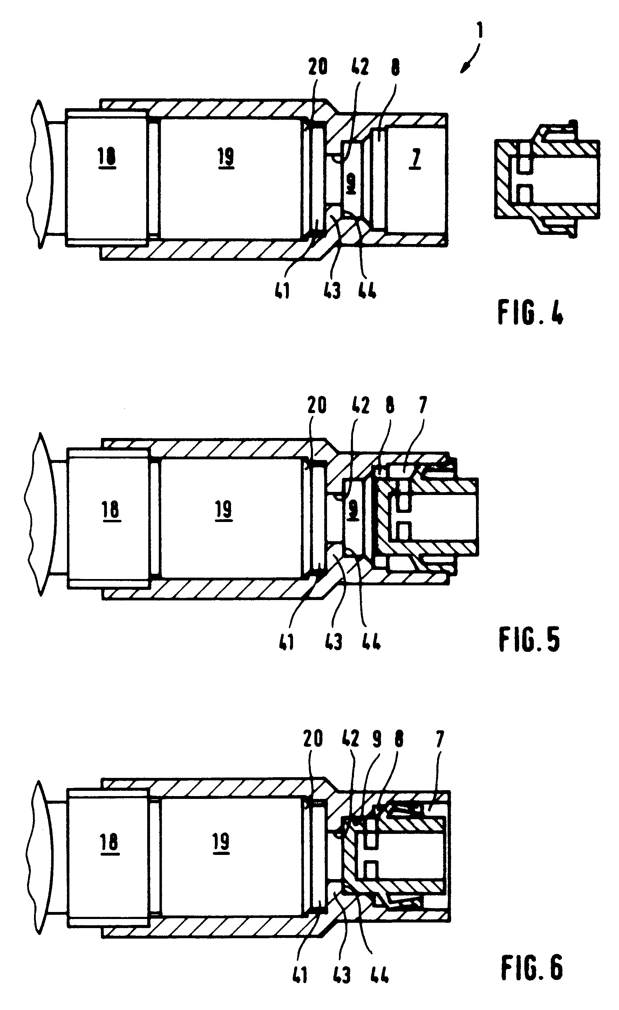

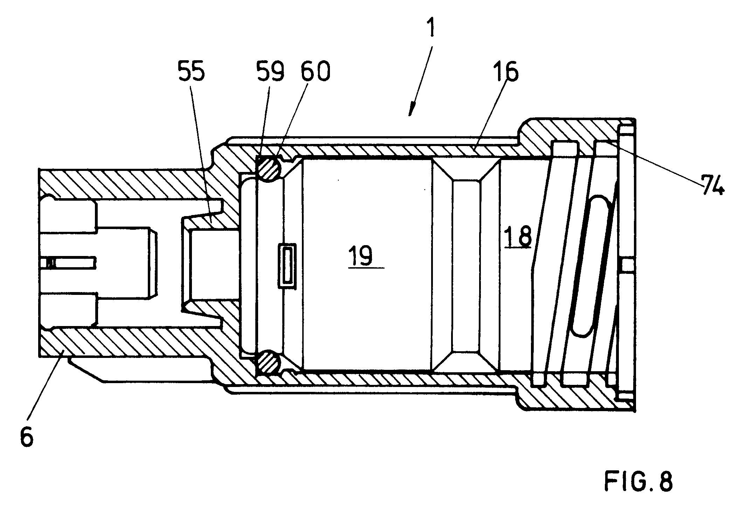

In den Figuren 4 bis 6 ist hingegen die Ausführungsform der erfindungsgemäßen Verschlußkappe 1 für die Dialysatseite dargestellt. Wie aus den Figuren 4 bis 6 ersichtlich ist, ist die Verschlußkappe für die Dialysatseite in ihren wesentlichen Teilen genauso ausgebildet wie die Verschlußkappe 1 für die Blutseite. Diesbezüglich kann daher auf die Erläuterungen zu den Figuren 1 bis 3 verwiesen werden.

Als Unterschied und zur Anpassung an die Dialysatseite des Dialysators ist zunächst hervorzuheben, daß der dritte Teilraum 20 des zweiten Abschnittes 16 einen Übergang zwischen dem zweiten Teilraum 19 und einem vierten Teilraum 41 bildet, der über eine zentrische Verbindungsausnehmung 42 mit der dritten Teilkammer 9 des ersten Abschnittes 6 verbunden ist. Hierbei ist die Verbindungsausnehmung 42 von einer umlaufenden nach innen zur Längsachse vorspringenden Anschlagwand 43 begrenzt. Die Anschlagwand 43 weist eine Dichtfläche 44 auf, die auf einer auf die dritte Teilkammer 9 zuweisenden Seite angeordnet ist.

Darüber hinaus wird aus Fig. 4 deutlich, daß der zweite Teilraum 19 in Axialrichtung länger ausgebildet ist als der zweite Teilraum 19 des ersten Kappenteiles 2 gemäß den Figuren 1 bis 3. Des weiteren verdeutlichen die Figuren 4 bis 6, daß das erste Kappenteil 2 bei dieser Ausbildung über eine Schraubverbindung mit dem entsprechenden Konnektoranschluß der Dialysatseite verbunden ist. Dies stellt jedoch keinen wesentlichen Unterschied dar, da ebenfalls die in den Figuren 1 bis 3 dargestellte Schnapp-Rastverbindung vorgesehen sein kann.

Die Figuren 5 und 6 zeigen wiederum die Offen- bzw. Schließstellung der Verschlußkappe 1. Diese Stellungen entsprechen im wesentlichen ebenfalls den Stellungen gemäß den Figuren 3 und 4. Die Dichtstelle befindet sich allerdings aufgrund des anders ausgebildeten ersten Kappenteiles 2, das kein Einsatzteil aufweist, zwischen der Dichtfläche 44 der Anschlagwand 43 und dem entsprechenden anliegenden Flächenbereich der Abschlußwand 33 des zweiten Kappenteiles 3. Dies ist im einzelnen der Darstellung der Fig. 6 zu entnehmen.

Ansonsten werden mit der Ausführungsform der erfindungsgemäßen Verschlußkappe 1 für die Dialysatseite die gleichen Effekte und Vorteile erreicht, wie diejenigen der Verschlußkappenausführung für die Blutseite, so daß auch diesbezüglich auf die entsprechenden Darlegungen im Zusammenhang mit den Figuren 1 bis 3 verwiesen werden kann.

Ergänzend ist zu den zusammenwirkenden Arretiereinrichtungen 45 und 46 am ersten Kappenteil 2 bzw. am Dialysatorflansch 4 zu sagen, daß dieser als Originalitätsverschluß ausgelegt werden kann. Hierzu ist es denkbar, eine Abreißsicherung vorzusehen, oder die als Nase ausgebildete Arretiereinrichtung 46 am Dialysatorflansch 4 mit einer exakt definierten Sollbruchstelle zu versehen.

Schließlich kann die Verschlußkappe 1 auch einstückig ausgebildet sein, sofern entsprechende spritzgußtechnische Vorkehrungen getroffen werden. Demzufolge können die beiden Kappenteile 2 und 3 miteinander über einen flexiblen Steg verbunden sein, der sowohl ein axiales Zusammenschieben der beiden Teile als auch im gewissen Maß eine radiale Verdrehung der beiden Teile zuläßt. Dieser nicht gezeigte Steg ist integral an den beiden Kappenteilen angeformt.

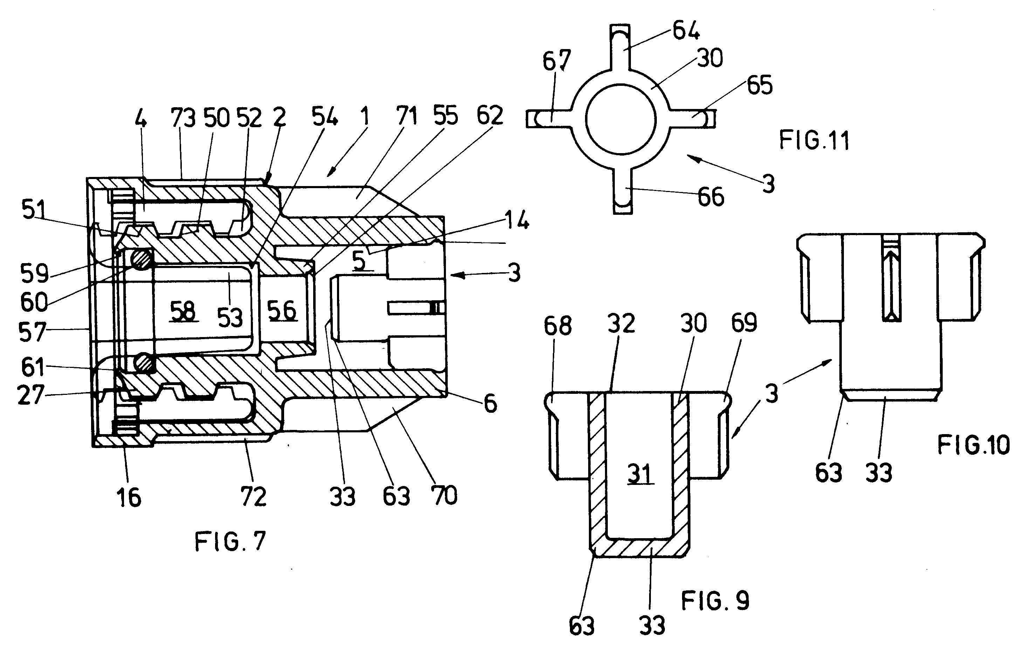

Gemäß Figur 7 ist eine zweite Ausführungsform einer erfindungsgemäßen Verschlußkappe für die Blutseite von Dialysatoren dargestellt, wobei alle Teile, die mit der vorhergehenden Ausführungsform übereinstimmen, mit den gleichen Bezugszeichen versehen wurden.

Demgemäß weist die zweite Ausführungsform der Verschlußkappe 1 ebenfalls ein erstes Kappenteil 2 und ein zweites stopfenartiges Kappenteil 3 auf.

Das erste Kappenteil 2 ist wiederum im wesentlichen rohrförmig ausgebildet, wobei eine besonders bevorzugte Ausführungsform kreiszylindrisch ist. Wie Fig. 7 verdeutlicht, ist die kreiszylindrische Begrenzungswand 27 des Kappenteiles 2 der zweiten Ausführungsform mit einem Außengewinde 50 versehen, das im Montagezustand mit einem Innengewinde 51 des nur teilweise dargestellten Dialysatorflansches 4 zusammenwirkt. Hierbei wird der äußere Dialysatorflansch 4 in einem langgestreckten Aufnahmeraum 53 aufgenommen, der zwischen dem äußeren zweiten kreiszylinderförmigen Abschnitt 16 und der innenliegenden Begrenzungswand 27 angeordnet ist.

Ein konzentrisch zum äußeren Dialysatorflansch 4 angeordneter innerer kreiszylinderförmiger Dialysatorbund 53 wird in einer konzentrisch angeordneten Aufnahmeausnehmung 54 des Abschnittes 16 angeordnet und reicht bis in den Bereich eines zylinderförmigen Fortsatzes 55, der mit einer Durchgangsausnehmung 56 versehen ist, die mit der Ausnehmung 54 des zweites Abschnittes 16 in Verbindung steht.

Auf die offene Stirnseite 57 zuweisend, weist die Begrenzungswand 27 eine kreisringförmig umlaufende Nut 59 auf, in der ein Dichtelement, vorzugsweise in Form eines O-Ringes 60 eingelegt ist. Wie Figur 7 verdeutlicht, legt sich der O-Ring im montierten Zustand so an den Dialysatorbund 53 und die Begrenzungswände der Nut 59 an, daß bei der Ausführungsform gemäß Figur 7 an dieser Stelle eine sterile Abdichtung des Dialysators bei aufgeschraubter Verschlußkappe 1 erfolgt. Fig. 7 zeigt hierbei, daß auf der Seite der offenen Stirnseite 57 die Nut 59 mit einem umlaufenden, nach innen zuweisenden Bund 61 versehen ist, der dazu dient, ein Herausrutschen des O-Ringes 60 aus der Nut 59 im nicht montierten Zustand der Verschlußkappe 1 zu verhindern.

Wie aus Fig. 7 ferner ersichtlich ist, ergibt sich eine Strömungsverbindung zwischen einem Strömungskanal 58, der innerhalb des Dialysatorbundes bzw. inneren Dialysatorflansches 53 angeordnet ist und der Durchgangsausnehmungen 56 des Fortsatzes 55. Die Durchgangsausnehmung 56 des Fortsatzes 55 wiederum steht bei der in Fig. 7 dargestellten Stellung des stopfenartigen Kappenteiles 3 mit dem Aufnahmeraum des ersten Abschnittes 6 des ersten Kappenteiles 2 in Verbindung, denn das stopfenartige Kappenteil 3 ist bei der in Fig. 7 dargestellten Stellung endseitig im Aufnahmeraum 5 angeorrdnet, so daß die abschlußwand 33 des zweiten Kappenteiles 3 einen Abstand zu einer ringförmigen Dichtfläche 62 aufnimmt, die kegelstumpfförmig angefast ist. Eine entsprechend ausgebildete außenseitige Dichtfläche 63 ist auf dem Außenrand der Abschlußwand 33 angeordnet.

Das Kappenteil 3 ist innerhalb der Aufnahmeausnehmung 5 über beispielsweise insgesamt vier Flügel 64 bis 67 geführt, deren Anordnung und Ausbildung insbesondere den Figuren 9 bis 11 entnommen werden kann. Die Flügel 64 bis 67 sind flächig ausgebildet und erstrecken sich von der Stirnseite 32 des Kappenteiles 3 bis ungefähr in dessen, in Längsrichtung gesehen, Mittelabschnitt. Wie Fig. 11 verdeutlicht, sind die Flügel 64 bis 67 jeweils um 90° beabstandet, um den Außenumfang des Kappenteiles 3 herum angeordnet und weisen an ihren radial äußeren Enden auf der Seite der Stirnseite 32 Vorsprünge auf, von denen in den Figuren 9 und 10 die Vorsprünge 68 und 69 sichtbar sind. Stirnseitig ist der erste Abschnitt 6 des Kappenteiles 2 mit einer sich nach außen erweiternden Anfasung 70 versehen, deren Form den Vorsprüngen der Flügel 64 bis 67 entspricht.

In der in Fig. 7 dargestellten Schaltstellung liegen die Vorsprünge der Flügel 64 bis 67 in dieser Anfasung 70′, so daß ein gewisser Widerstand beim Eindrücken des zweiten Kappenteiles 3 in Richtung auf den Fortsatz 55 überwunden werden muß. Durch die radial sich nach außen erstreckende Anordnung der Flügel 64 bis 67 wird das zweite Kappenteil 3 im Aufnahmeraum 5 zentriert und kann von der in Fig. 7 dargestellten Stellung in eine Stellung in den Innenraum hinein verschoben werden, in der sich Dichtflächen 62 und 63 aufeinander auflegen, wodurch eine Abdichtung der Durchgangsausnehmung 56 erreicht wird, so daß der Dialysator hermetisch abgeschlossen ist. Bei Zurückbewegen in die in Fig. 7 dargestellte Stellung hingegen wird die Strömungsverbindung zwischen der Durchgangsausnehmung 56 und dem Aufnahmeraum 5 wieder hergestellt, wobei aufgrund der Anordnung der Flügel 64 bis 67 in jeweiligen 90° Abständen am Außenumfang des Kappenteiles 3 zwischen den Flügeln und der inneren Begrenzungswand 14 ebenfalls ein Strömungsquerschnitt freigegeben ist.

Zu der Ausführungsform des Kappenteiles 2 der Verschlußkappe 1 gemäß Figur 7 ist nachzutragen, daß diese auf dem ersten und zweiten Abschnitt 6 bzw. 16 jeweils mit einer Mehrzahl von nach außen weisenden Verstärkungsrippen versehen sein kann, von denen in Fig. 7 die Verstärkungsrippen 70 bis 73 sichtbar sind. Hierbei sind die Verstärkungsrippen 70 und 71 am ersten Abschnitt 6 soweit radial nach außen gezogen, daß sie ungefähr dem Außendurchmesser des zweiten Abschnittes 16 entsprechen. Zwischen den Rippen 70 und 71 kann eine Mehrzahl weiterer Rippen mit radial geringeren Abmessungen vorgesehen sein.

Ansonsten wird bezüglich aller übereinstimmenden Merkmale zwischen den Ausführungsformen gemäß den Fig. 1 bis 3 und derjenigen gemäß den Figuren 7 bis 11 auf die voranstehenden Darlegungen verwiesen.

In Figur 8 ist schließlich ein Kappenteil 1 für die Dialysatseite dargestellt, das in seinen wesentlichen Merkmalen mit demjenigen Kappenteil gemäß der Ausführungsform der Figur 7 übereinstimmt. Mithin ist hier ebenfalls eine Abdichtung mittels elastischer Dichteinrichtung, vorzugsweise in Form eines O-Ringes 60 vorgenommen, und es wird ein zweites Kappenteil 3 verwendet, das demjenigen der Fig. 9 bis 11 entspricht. Da kann diesbezüglich auf die Erläuterungen zu den Figuren 7 und 9 bis 11 Bezug genommen werden.

Aufgrund der andersartigen Ausbildung des dialysatseitigen Flansches des Dialysators, ist das Kappenteil 1 auf der Dialysatseite entsprechend den konstruktiven Abänderungen gemäß der Ausführungsform der Figuren 4 bis 6 ausgebildet und weist mithin vor allem ein sich an den ersten Abschnitt anschließenden verlängerten zweiten Abschnitt 16 auf, im Bereich dessen stirnseitiger Öffnung ein Innengewinde 74 zum Zusammenwirken mit einem Außengewinde auf dem dialysatseitigen Flansch des Dialysators angeordnet ist. Ferner ist als Unterschied zu dem Kappenteil gemäß Fig. 7 hervorzuheben, daß der O-Ring 60 dem Fortsatz 55 näherliegend angeordnet ist, jedoch ebenfalls in einer Nut 59 liegt, so daß sich beim Aufschrauben des dialysatseitigen Kappenteiles 1, das in Fig. 8 in seiner offenen Stellung dargestellt ist, ebenfalls eine sterile Abdichtung durch die O-Ring-Dichtung 60 ergibt.

Bezüglich aller sonstigen Gemeinsamkeiten kann wiederum auf die Ausführungsform gemäß den Figuren 4 und 6 bzw. die Gemeinsamkeiten mit dem Kappenteil 1 gemäß Figur 7 verwiesen werden.

## Patentansprüche

1. Verschlußkappe (1) für Dialysatoren mit ersten und zweiten Kappenteilen (2, 3), von denen das erste Kappenteil (2) an einem Dialysatorflansch (4) anbringbar ist, während das zweite Kappenteil (3) zwischen einer Offenstellung, in der der Durchtritt eines Sterilisationsmediums erfolgt, und einer Schließstellung bewegbar ist, dadurch gekennzeichnet, daß das erste Kappenteil (2) einen Anschlußabschnitt (16) mit einer Dichteinrichtung (22, 60), die den Dialysatorflansch (4) im Einbauzustand steril dichtet, und ein rückwärtiges Teil (6) aufweist, das mit einer In-line-Sterilisationseinrichtung in Strömungsverbindung bringbar ist.

2. Verschlußkappe nach Anspruch 1, dadurch gekennzeichnet, daß das erste Kappenteil (2) im wesentlichen rohrförmig ausgebildet ist und an einer Seite einen Aufnahmeraum (5) für das zweite Kappenteil (3) aufweist, der in einem ersten Abschnitt bzw. dem rückwärtigen Teil (6) angeordnet ist, und daß der Aufnahmeraum (5) drei aufeinanderfolgende Teilkammern (7, 8, 9) aufweist, die von der offenen Stirnseite (10) her mit abnehmenden Durchmessern zu einem der Stirnseite (10) gegenüberliegenden Endbereich (11) des ersten Abschnittes (6) in diesem angeordnet sind.

3. Verschlußkappe nach Anspruch 2, dadurch gekennzeichnet, daß zwischen der ersten Teilkammer (7) mit dem größten Durchmesser und der zweiten Teilkammer (8) eine im wesentlichen senkrecht zur Längsachse des ersten Kappenteils (2) nach innen verlaufende Stufe (12) angeordnet ist, und daß die zweite Teilkammer (8) und die dritte Teilkammer (9) kleinsten Durchmessers über einen kegelförmigen Zwischenabsatz (13) miteinander verbunden sind.

4. Verschlußkappe nach Anspruche 2 oder 3, dadurch gekennzeichnet, daß die Innenfläche (14) der ersten Teilkammer (7) eine Führungs- und Haltefläche für das zweite Kappenteil (3) bildet.

5. Verschlußkappe nach einem der Ansprüche 2 bis 4, dadurch gekennzeichnet, daß die Innenfläche (15) der dritten Teilkammer (9) ebenfalls eine Führungsfläche für das zweite Kappenteil (3) bildet.

6. Verschlußkappe nach einem der Ansprüche 1 bis 5, dadurch gekennzeichnet, daß der Anschlußabschnitt (16) mit dem ersten Abschnitt (6) anschließend an einen Endbereich (11) desselben verbunden ist und sein Außendurchmesser größer ist als der Außendurchmesser des ersten Abschnittes (6), und daß der Anschlußabschnitt (16) eine offene Stirnseite (17) aufweist, an die sich nach innen drei Teilräume (18, 19, 20) anschließen.

7. Verschlußkappe nach Anspruch 6, dadurch gekennzeichnet, daß der unmittelbar an die Stirnseite (17) anschließende Teilraum (18) einen größeren Innendurchmesser als der mittlere Teilraum (19) aufweist und mit diesem über eine im wesentlichen senkrecht zur Längsachse des ersten Kappenteils (2) angeordnete Stufe (21) verbunden ist.

8. Verschlußkappe nach Anspruch 1 oder 6 oder 7, dadurch gekennzeichnet, daß die Innenfläche (22) des zweiten Teilraumes (19) eine Führungsfläche und die Dichteinrichtung für den Dialysatorflansch (4) bildet.

9. Verschlußkappe nach einem der Ansprüche 6 bis 8, dadurch gekennzeichnet, daß der dritte Teilraum (20) eine kegelstumpfförmige Innenfläche (23) aufweist, die einen stetigen Übergang zwischen dem zweiten Teilraum (19) und der dritten Teilkammer (9) schafft, und daß innerhalb der Teilräume (18 bis 20) konzentrisch zum zweiten Abschnitt (16) ein topfförmiges Einsatzteil (24) festgelegt ist, dessen Außenfläche (25) mit den Innenflächen der Teilräume einen freien Strömungsdurchgang begrenzt, der eine Strömungsverbindung zwischen der offenen Stirnseite (17) des zweiten Abschnittes (16) und der dritten Teilkammer (9) des ersten Abschnittes (6) schafft.

10. Verschlußkappe nach Anspruch 9, dadurch gekennzeichnet, daß das Einsatzteil (24) eine offene Stirnseite (26) aufweist, die vorzugsweise in einer Ebene mit der offenen Stirnseite (17) liegt, und daß das Einsatzteil (24) vorzugsweise eine kreiszylindrische Begrenzungswand (27) aufweist, die an einer der offenen Stirnseite (26) gegenüberliegenden Seite mit einer Abschlußwand (29) verbunden ist.

11. Verschlußkappe nach Anspruch 10, dadurch gekennzeichnet, daß die Abschlußwand (28) eine auf die dritte Teilkammer (9) hinweisende Außenfläche (29) aufweist.

12. Verschlußkappe nach einem der Ansprüche 1 bis 11, dadurch gekennzeichnet, daß das zweite Kappenteil (3) topfförmig ausgebildet ist und eine um die Längsachse herum verlaufende Wand (30) aufweist, die einen Innenraum (31) begrenzt und daß der Innenraum (31) an einer Stirnseite (32) offen ist, und an der gegenüberliegenden Stirnseite von einer im wesentlichen senkrecht zur Längsachse angeordneten Abschlußwand (33) geschlossen ist, wobei die Wand (30) zumindestens eine Verbindungsöffnung (34 oder 35) aufweist, die den Innenraum (31) mit der Außenwandfläche (36) verbindet.

13. Verschlußkappe nach Anspruch 12, dadurch gekennzeichnet, daß auf der Außenwandfläche (36) eine nach außen weisende Führungs- und Halteeinrichtung (37) angeordnet ist.

14. Verschlußkappe nach Anspruch 13, dadurch gekennzeichnet, daß die Führungs- und Halteeinrichtung (37) einen im Abstand zur Außenwandfläche (36) angeordneten elastisch nachgiebigen Führungsring (38) und einen von diesem nach außen weisenden Anschlagring (39) aufweist, und daß der Führungsring (38) über einen Bund (40) mit der Wand (30) verbunden ist.

15. Verschlußkappe nach einem der Ansprüche 1 bis 7, dadurch gekennzeichnet, daß der dritte Teilraum (20) einen Übergang zwischen dem zweiten Teilraum (19) und einem vierten Teilraum (41) bildet, der über eine zentrische Verbindungsausnehmung (42) mit der dritten Teilkammer (9) verbunden ist, und daß die Verbindungsausnehmung (42) von einer umlaufenden, nach innen zur Längsachse vorspringenden Anschlagwand (43) begrenzt ist.

16. Verschlußkappe nach Anspruch 15, dadurch gekennzeichnet, daß die Anschlagwand (43) eine auf die dritte Teilkammer (9) zuweisende Dichtfläche (44) aufweist.

17. Verschlußkappe nach einem der Ansprüche 1 bis 16, dadurch gekennzeichnet, daß das erste Kappenteil (2) mit einer Arretiereinrichtung (45) versehen ist, die mit einer komplementären Arretiereinrichtung (46) am Dialysatorflansch (4) im montierten Zustand des zweiten Kappenteils (2) zusammenwirkt.

18. Verschlußkappe nach Anspruch 17, dadurch gekennzeichnet, daß die zusammenwirkenden Arretiereinrichtungen (45, 46) als Schnapp-, Rast-, Schraub- oder Bajonettverschluß ausgebildet sind.

19. Verschlußkappe nach Anspruch 17 oder 18, dadurch gekennzeichnet, daß die Arretiereinrichtungen (45, 46) als Originalitätsverschluß ausgebildet sind.

20. Verschlußkappe nach Anspruch 19, dadurch gekennzeichnet, daß die Arretiereinrichtung (46) am Dialysatorflansch (4) als Nasenvorsprung ausgebildet ist, der mit einer definierten Sollbruchstelle versehen ist.

21. Verschlußkappe nach Anspruch 1 oder 2, dadurch gekennzeichnet, daß das erste Kappenteil (2) eine elastische Dichteinrichtung (60) aufweist.

22. Verschlußkappe nach Anspruch 21, dadurch gekennzeichnet, daß die elastische Dichteinrichtung als O-Ring (60) ausgebildet ist, der in einer ringförmig umlaufenden inneren Dichtnut (59) angeordnet ist und im Montagezustand mit einem Dialysatorbund (53) des Dialysatorflansches (4) zur Erzeugung der Dichtwirkung zusammenwirkt.

23. Verschlußkappe nach Anspruch 21 oder 22, dadurch gekennzeichnet, daß im Aufnahmeraum (5) ein zylindrischer Fortsatz (55) angeordnet ist, der eine innere Durchgangsausnehmung (56) aufweist, die mit dem Aufnahmeraum (5) in Offenstellung des Kappenteils (3) in Strömungsverbindung steht.

24. Verschlußkappe nach Anspruch 23, dadurch gekennzeichnet, daß der Fortsatz (55) eine ringförmige Dichtfläche (62) aufweist, die mit einer komplementär ausgebildeten ringförmigen Dichtfläche (63) des zweiten Kappenteiles (3) in dessen Schließstellung zusammenwirkt.

25. Verschlußkappe nach einem der Ansprüche 21 bis 24, dadurch gekennzeichnet, daß das zweite Kappenteil (3) vorzugsweise vier in einem Winkel von 90° angeordnete Flügel (64 bis 67) aufweist, die umfangsseitig auf der Außenwand (30) des Kappenteiles (3) angeordnet sind, und daß die Flügel (64 bis 67) mit Vorsprüngen (68, 69) versehen sind, die radial nach außen vom Außenrand der Flügel (64 bis 67) vorspringen.

26. Verschlußkappe nach einem der Ansprüche 21 bis 25, dadurch gekennzeichnet, daß die blutseitige Verschlußkappe (1) mit einem in einem Aufnahmeraum (52) angeordneten Außengewinde (50) zum Zusammenwirken mit einem Innengewinde (51) des Dialysatorflansches (4) versehen ist.

27. Verschlußkappe nach einem der Ansprüche 21 bis 26, dadurch gekennzeichnet, daß die dialysatseitige Verschlußkappe (1) mit einem Innengewinde (74) zum Zusammenwirken mit einem Außengewinde auf dem dialysatseitigen Dialysatorflansch versehen ist.

## Claims

1. Closure cap (1) for dialyzers comprising first and second cap parts (2, 3), of which the first cap part (2) is adapted to be attached to a dialyzer flange (4) whilst the second cap part (3) is movable between an open position in which the passage of a sterilization medium takes place and a closure position, characterized in that the first cap part (2) comprises a connection portion (16) having a sealing means (22, 60), which seals the dialyzer flange (4) in the assembled state in sterile manner, and a rearward portion (6) which is adapted to be brought into flow connection with an in-line sterilization means.

2. Closure cap according to claim 1, characterized in that the first cap part (2) is made substantially tubular and comprises at one side a receiving compartment (5) for the second cap part (3) which is arranged in a first portion or the rearward portion (6) and that the receiving compartment (5) comprises three consecutive subchambers (7, 8, 9) which are arranged from the open end side (10) with decreasing diameters up to an end region (11) of the first portion (6) opposite the end side (10) in said portion.

3. Closure cap according to claim 2, characterized in that between the first subchamber (7) with the greatest diameter and the second subchamber (8) a step (12) extending inwardly substantially perpendicularly to the longitudinal axis of the first cap part (2) is arranged and that the second subchamber (8) and the third subchamber (9) of smallest diameter are connected together via a conical intermediate portion (13).

4. Closure cap according to claim 2 or 3, characterized in that the inner surface (14) of the first subchamber (7) forms a guide and retaining surface for the second cap part (3).

5. Closure cap according to any one of claims 2 to 4, characterized in that the inner surface (15) of the third subchamber (9) also forms a guide surface for the second cap part (3).

6. Closure cap according to any one of claim 1 to 5, characterized in that the connection portion (16) is connected to the first portion (6) following an end region (11) thereof and its outer diameter is greater than the outer diameter of the first portion (6) and the connection portion (16) has a opened end side (17) adjoined inwardly by three subspaces (18, 19, 20).

7. Closure cap according to claim 6, characterized in that the subspace (18) immediately adjoining the end side (17) has a greater inner diameter than the middle subspace (19) and is connected to the latter via a step (21) arranged substantially perpendicularly to the longitudinal axis of the first cap part (2).

8. Closure cap according to claim 1 or 6 or 7, characterized in that the inner surface (22) of the second subspace (19) forms a guide surface and the sealing means for the dialyzer flange (4).

9. Closure cap according to any one of claims 6 to 8, characterized in that the third subspace (20) comprises a frustoconical inner surface (23) which provides a gradual transition between the second subspace (19) and the third subchamber (9) and that within the subspaces (18 to 20) concentrically to the second portion (16) a pot-shaped insert member (24) is fixed whose outer surface (25) defines with the inner surface of the subspaces a free flow passage which creates a flow connection between the open end side (17) of the second portion (16) and the third subchamber (9) of the first portion (6).

10. Closure cap according to claim 9, characterized in that the insert member (24) has an open end side (26) which lies preferably in a plane with the open end side (17) and that the insert member (24) preferably comprises a cylindrical boundary wall (27) which is connected at a side opposite the open end side (26) to a closure wall (29).

11. Closure cap according to claim 10, characterized in that the closure wall (28) comprises an outer surface (29) directed towards the third subchamber (9).

12. Closure cap according to any one of claims 1 to 11, characterized in that the second cap part (3) is made pot-shaped and comprises a wall (30) which extends round the longitudinal axis and defines the inner space (31) and that the inner space (31) is open at an end side (32) and at the opposite end side is closed by a closed by a closure wall (33) arranged substantially perpendicularly to the longitudinal axis, with the wall (30) comprising at least one connection opening (34 or 35) which connects the inner space (31) to the outer wall surface (36).

13. Closure cap according to claim 12, characterized in that on the outer wall surface (36) an outwardly directing guide and retaining means (37) is arranged.

14. Closure cap according to claim 13, characterized in that the guide and retaining means (37) comprises a resiliently yielding guide ring (38) arranged spaced from the outer wall surface (36) and a stop ring (39) directed outwardly from said guide ring (38) and that the guide ring (38) is connected via a collar (40) to the wall (30).

15. Closure cap according to any one of claims 1 to 7, characterized in that the third subspace (20) forms a transition between the second subspace (19) and a fourth subspace (41) which is connected via a central connection recess (42) to the third subchamber (9) and that the connection recess (42) is defined by an encircling stop wall (43) projecting inwardly to the longitudinal axis.

16. Closure cap according to claim 15, characterized in that the stop wall (43) comprises a sealing surface (44) directed towards the third subchamber (9).

17. Closure cap according to any one of claims 1 to 16, characterized in that the first cap part (2) is provided with an arresting means (45) which cooperates with a complementary arresting means (46) on the dialyzer flange (4) in the mounted state of the second cap part (2).

18. Closure cap according to claim 17, characterized in that the cooperating arresting means (45, 46) are formed as snap-action, detent, screw or bayonet fastener.

19. Closure cap according to claim 17 or 18, characterized in that the arresting means (45, 46) are formed as intactness closure.

20. Closure cap according to claim 19, characterized in that the arresting means (46) on the dialyzer flange (4) is formed as nose projection which is provided with a defined intended breakage point.

21. Closure cap according to claim 1 or 2, characterized in that the first cap part (2) comprises an elastic sealing means (60).

22. Closure cap according to claim 21, characterized in that the elastic sealing means is formed as O-ring (60) which is arranged in an annularly encircling inner sealing groove (59) and in the assembled state cooperates with a dialyzer collar (53) of the dialyzer flange (4) for generating the sealing effect.

23. Closure cap according to claim 21 or 22, characterized in that in the receiving compartment (5) a cylindrical extension (55) is arranged which comprises an inner passage recess (56) which is in flow connection with the receiving compartment (5) in the open position of the cap part (3).

24. Closure cap according to claim 23, characterized in that the extension (55) comprises an annular sealing surface (62) which cooperates with a complementarily formed annular sealing surface (63) of the second cap part (3) in the closure position thereof.

25. Closure cap according to any one of claims 21 to 24, characterized in that the second cap part (3) referably comprises four vanes (64 to 67) which are arranged at an angle of 90° and disposed circumferentially on the outer wall (30) of the cap part (3) and that the vanes (64 to 67) are provided with projections (68, 69) which project radially outwardly from the outer edge of the vanes (64 to 67).

26. Closure cap according to any one of claims 21 to 25, characterized in that the blood-side closure cap (1) is provided with an outer thread (50) arranged in a receiving compartment (52) for cooperating with an inner thread (51) of the dialyzer flange (4).

27. Closure cap according to any one of claims 21 to 26, characterized in that the dialyzate-side closure cap (1) is provided with an inner thread (74) for cooperation with an outer thread on the dialyzate-side dialyzer flange.

## Revendications

1. Capuchon de fermeture (1) pour dialyseurs comprenant une première et une seconde partie (2, 3) dont la première partie (2) peut être montée sur une bride (4) d'un dialyseur, tandis que la seconde partie (3) peut être déplacée entre une position d'ouverture, dans laquelle le passage pour un agent de stérilisation est établi, et une position de fermeture, caractérisé en ce que la première partie (2) du capuchon présente une section de raccordement (16) avec un dispositif d'étanchéité (22, 60), qui assure l'étanchéité stérile de la bride (4) du dialyseur à l'état monté en place et une partie arrière (6) qui peut être amenée en communication d'écoulement avec un dispositif de stérilisation en ligne.

2. Capuchon de fermeture selon la revendication 1, caractérisé en ce que la première partie (2) est en substance tubulaire et présente, d'un côté, un espace récepteur (5) pour la seconde partie (3) du capuchon, qui est amenagé dans une première section ou dans la partie arrière (6), et l'espace récepteur (5) présente trois chambres partielles successives (7, 8, 9), qui sont aménagées dans cet espace récepteur avec des diamètres décroissants à partir de la face d'about ouverte (10) vers une zone d'extrémité (11) opposée à la face d'about (10) de la première section (6).

3. Capuchon de fermeture selon la revendication 2, caractérisé en ce qu'entre la première chambre partielle (7) du plus grand diamètre et la deuxième chambre partielle (8) est prévu un gradin (12) s'étendant vers l'intérieur et sensiblement perpendiculaire à l'axe longitudinal de la première partie (2) du capuchon, et la deuxième chambre partielle (8) ainsi que la troisième chambre partielle (9) du plus petit diamètre sont reliées l'une à l'autre par un épaulement intermédiaire (13) de forme conique.

4. Capuchon de fermeture selon la revendication 2 ou 3, caractérisé en ce que la surface interne (14) de la première chambre partielle (7) forme une surface de guidage et de retenue pour la seconde partie (3) du capuchon.

5. Capuchon de fermeture selon l'une quelconque des revendications 2 à 4, caractérisé en ce que la surface interne (15) de la troisième chambre partielle (9) forme également une surface de guidage pour la seconde partie (3) du capuchon.

6. Capuchon de fermeture selon l'une quelconque des revendications 1 à 5, caractérisé en ce que la section de raccordement (16) est reliée à la première section (6), à proximité d'une zone d'extrémité (11) de celle-ci, et son diamètre externe est plus grand que le diamètre externe de la deuxième section (6), et la section de raccordement (16) présente une face d'about ouverte (17) à laquelle se raccordent vers l'intérieur trois espaces partiels (18, 19, 20).

7. Capuchon de fermeture selon la revendication 6, caractérisé en ce que l'espace partiel (18) se raccordant directement à la face d'about (17) présente un plus grand diamètre interne que l'espace partiel médian (19) et est raccordé à celui-ci via un gradin (21) en substance perpendiculaire à l'axe longitudinal de la première partie (2) du capuchon.

8. Capuchon de fermeture selon la revendication 1 ou 6 ou 7, caractérisé en ce que la surface interne (22) du deuxième espace partiel (19) forme une surface de guidage et le dispositif d'étanchéité pour la bride (4) du dialyseur.

9. Capuchon de fermeture selon l'une quelconque des revendications 6 à 8, caractérisé en ce que le troisième espace partiel (20) présente une surface interne tronconique (23) qui assure une transition progressive entre le deuxième espace partiel (19) et la troisième chambre partielle (9), et, qu'à l'intérieur des espaces partiels (18 à 20), concentriquement à la seconde section (16), est fixée une pièce rapportée en forme de pot (24), dont la surface externe (25) délimite, avec les surfaces internes des espaces partiels, un passage d'écoulement libre qui établit une communication d'écoulement entre la face d'about ouverte (17) de la seconde section (16) et la troisième chambre partielle (9) de la première section (6).

10. Capuchon de fermeture selon la revendication 9, caractérisé en ce que la pièce rapportée (24) présente une face d'about ouverte (26), qui se trouve de préférence dans un plan avec la face d'about ouverte (17), et la pièce rapportée (24) comporte de préférence une paroi de délimitation (27) en forme de cylindre circulaire, qui est reliée d'un côté opposé à la face d'about ouverte (26) à une paroi de fermeture (28).

11. Capuchon de fermeture selon la revendication 10, caractérisé en ce que la paroi de fermeture (28) présente une face externe (29) tournée vers la troisième chambre partielle (9).

12. Capuchon de fermeture selon l'une quelconque des revendications 1 à 11, caractérisé en ce que la seconde partie (3) du capuchon a la forme d'un pot et présente une paroi (30) s'étendant autour de l'axe longitudinal, qui délimite un espace interne (31), et l'espace interne (31) est ouvert à une face d'about (32) et est fermé à la face d'about opposée par une paroi de fermeture (33) en substance perpendiculaire à l'axe longitudinal, la paroi (30) présentant au moins une ouverture de communication (34 ou 35) qui raccorde l'espace interne (31) à la surface de paroi externe (36).

13. Capuchon de fermeture selon la revendication 12, caractérisé en ce qu'un dispositif de guidage et de retenue (37) tourné vers l'extérieur est prévu sur la surface de paroi externe (36).

14. Capuchon de fermeture selon la revendication 13, caractérisé en ce que le dispositif de guidage et de retenue (37) comporte un anneau de guidage (38) élastiquement flexible, disposé à distance de la surface de paroi externe (36) et un anneau d'arrêt (39) orienté vers l'extérieur de celui-ci, et l'anneau de guidage (38) est relié à la paroi (30) par une collerette (40).

15. Capuchon de fermeture selon l'une quelconque des revendications 1 à 7, caractérisé en ce que le troisième espace partiel (20) forme une transition entre le deuxième espace partiel (19) et un quatrième espace partiel (41) qui est raccordé à la troisième chambre partielle (9) via un évidement de communication central (42), et l'évidement de communication (42) est délimité par une paroi d'arrêt (43) périphérique faisant saillie vers l'intérieur vers l'axe longitudinal.

16. Capuchon de fermeture selon la revendication 15, caractérisé en ce que la paroi d'arrêt (43) présente une surface d'étanchéité (44) tournée vers la troisième chambre partielle (9).

17. Capuchon de fermeture selon l'une quelconque des revendications 1 à 16, caractérisé en ce que la première partie (2) du capuchon est dotée d'un dispositif d'arrêt (45), qui coopère avec un dispositif d'arrêt complémentaire (46) prévu sur la bride (4) du dialyseur lorsque la seconde partie (2) du dialyseur est montée en place.

18. Capuchon de fermeture selon la revendication 17, caractérisé en ce que les dispositifs d'arrêt coopérants (45, 46) forment une fermeture à déclic, à crans, à vis ou à baïonnette.

19. Capuchon de fermeture selon la revendication 17 ou 18, caractérisé en ce que les dispositifs d'arrêt (45, 46) forment une fermeture antieffraction.

20. Capuchon de fermeture selon la revendication 19, caractérisé en ce que le dispositif d'arrêt (46) a la forme d'une saillie sur la bride du dialyseur (4), laquelle saillie est dotée d'un point de rupture défini.

21. Capuchon de fermeture selon la revendication 1 ou 2, caractérisé en ce que la première partie (2) du capuchon présente un dispositif d'étanchéité élastique (60).

22. Capuchon de fermeture selon la revendication 21, caractérisé en ce que le dispositif d'étanchéité élastique a la forme d'un joint torique (60) qui est disposé dans une gorge d'étanchéité interne annulaire (59) et coopère avec un embout (53) de la bride (4) du dialyseur pour produire l'effet d'étanchéité.

23. Capuchon de fermeture selon la revendication 21 ou 22, caractérisé en ce que dans l'espace récepteur (5) est disposé un prolongement cylindrique (55) qui présente un évidement de passage (56) en communication d'écoulement avec l'espace récepteur (5) dans la position d'ouverture de la partie (3) du capuchon.

24. Capuchon de fermeture selon la revendication 23, caractérisé en ce que le prolongement (55) présente une surface d'étanchéité annulaire (62) qui coopère avec une surface d'étanchéité (63) annulaire de forme complémentaire de la seconde partie (3) du capuchon, dans la position de fermeture de celle-ci.

25. Capuchon de fermeture selon l'une quelconque des revendications 21 à 24, caractérisé en ce que la seconde partie (3) du capuchon comporte de préférence quatre ailettes (64 à 67) agencées à intervalles de 90°, qui sont prévues sur la paroi externe (30) de la partie (3) du capuchon, et les ailettes (64 à 67) sont dotées de saillies (68, 69) qui s'étendent radialement vers l'extérieur par rapport au bord externe des ailettes (64 à 67).

26. Capuchon de fermeture selon l'une quelconque des revendications 21 à 25, caractérisé en ce que le capuchon de fermeture côté sang (1) est doté d'un filet extérieur (50) disposé dans un espace récepteur (52) et destiné à coopérer avec un filet intérieur (51) de la bride (4) du dialyseur.

27. Capuchon de fermeture selon l'une quelconque des revendications 21 à 26, caractérisé en ce que le capuchon de fermeture (1) côté dialysat est doté d'un filet intérieur (74) destiné à coopérer avec un filet extérieur prévu sur la bride du dialyseur côté dialysat.
